Europäisches Patentamt

(19) European Patent Office

Office européen des brevets

(11) Publication number: 0 176 351
A2

(12) # EUROPEAN PATENT APPLICATION

(21) Application number: 85306793.2

(22) Date of filing: 24.09.85

(51) Int. Cl.⁴: **A 61 B 17/28**

(30) Priority: 26.09.84 IL 73079

(43) Date of publication of application:
02.04.86 Bulletin 86/14

(84) Designated Contracting States:
AT BE CH DE FR GB IT LI LU NL SE

(71) Applicant: Porat, Michael
52 Hamitnadev Street
Tel Aviv 69690(IL)

(71) Applicant: Porat, Amir
22 Rachvat Eilan Street
Givat Shaul Ramat Eilan(IL)

(72) Inventor: Porat, Michael
52 Hamitnadev Street
Tel Aviv 69690(IL)

(72) Inventor: Porat, Amir
22 Rachvat Eilan Street
Givat Shaul Ramat Eilan(IL)

(74) Representative: Fisher, Bernard et al,
Raworth, Moss & Cook 36 Sydenham Road
Croydon Surrey CR0 2EF(GB)

(54) **Gripper means for medical instruments.**

(57) Gripper means on each of a pair of gripping parts (1) of a gripping tool, particularly a medical instrument, wherein the teeth (3) and the interstices (5) on one side of an imaginary longitudinal median line (2) form a surface relief pattern which is substantially duplicated by the teeth (4) and interstices (6) on the other side of the line (2), but in the reverse sense, so that imaginary folding of the whole surface along the median line (2) will result in a substantial mating fit between teeth (3, 4) on one side of the line (2) and interstices (5, 6) on the other side of the line (2).

Preferably the teeth (3, 4) extend at the same angle on the two sides of the longitudinal median line (2) of the part, the teeth (3) on one side being staggered relative to those (4) on the other side.

Desirably the gripping parts (1) are of substantially identical construction so that a substantial mating fit is provided between the teeth (3, 4) and interstices (5, 6) of one of the pair of gripping parts (1) with the corresponding teeth and interstices of the other gripping part when the two gripping parts are brought together in use.

## Gripper Means for Medical Instruments

The present invention concerns the gripper means on gripping tools, especially medical instruments such as forceps and like medical instruments, e.g. clamps, tongs, tweezers and pincers, used in medicine, hereinafter collectively referred to as "forceps".

Forceps and the like medical instruments have been made of metal, the provision of the gripper means thereon being effected by special operating procedures such as milling, stamping or the like. Since this is a costly process, plastics forceps have come into use in which the gripper means can be cast together with the entire instrument. However, for each instrument usually two separate moulds have to be provided so that when an instrument is assembled from two parts, the gripper means will engage each other.

The present invention seeks to provide a gripper means for metal or plastics gripping tools, which permits interengagement of two identical parts to make one instrument.

Accordingly, the present invention is characterised in that the teeth and the interstices on one side of an imaginary longitudinal median line form a surface relief pattern which is substantially duplicated by the teeth and interstices on the other side of the line, but in the reverse sense, so that imaginary folding of the whole surface along the median line will result in a substantial mating fit between teeth on one side of the line and interstices on the other side of the line.

Preferably the teeth and the interstices cover substantially the whole of the inside surface of the gripping part, wherein the teeth and the interstices on each side of the median line are arranged in straight, parallel rows.

In its preferred form, the invention consists of gripper means on the gripping parts of a gripping tool comprising teeth which extend at the same angle on the two sides of an imaginary longitudinal median line of each part, the teeth on one side being staggered relative to those on the other. In this manner when assembling the two parts of the tool with the gripper means facing each other, the teeth on one side of the median line will engage in the interstices between the teeth on the same side.

It has been found that the gripping action of this arrangement is much more forceful than that of any of the gripper means known up until now.

In the following description, particular reference will be made to the use of the present invention in the construction of a medical instrument. It will be appreciated by those skilled in the art that the general principle underlying the present invention, although applicable to any gripping tool, e.g. household pliers or manicure tweezers, is particularly suitable for application to medical instruments where good gripping of veins, delicate threads, hair, body tissue and the like is required. Furthermore, where medical instruments are "disposable", i.e. intended to be used only once, the mass-production at low cost of two identical parts which can be joined together by, e.g. welding, is a significant advantage.

0176351

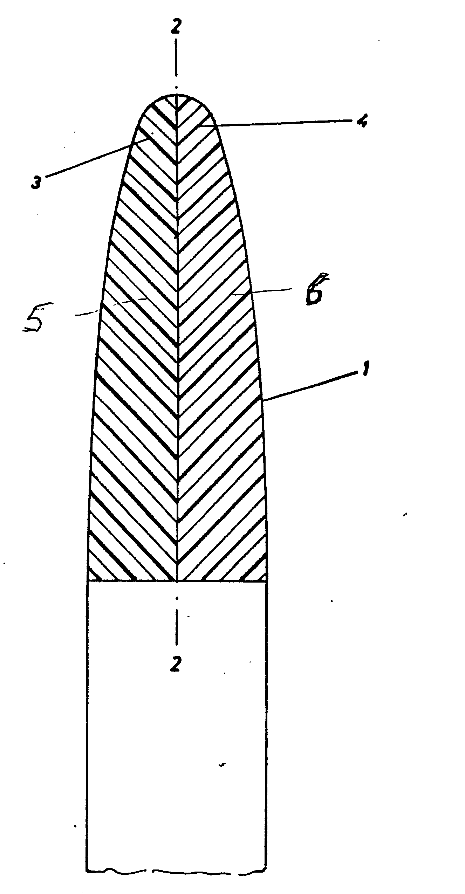

The invention is illustrated by way of example only in the single figure of the accompanying drawing showing a schematic plan view of the gripping part of a forceps according to the invention.

The tip or gripping part 1 of one of the pair of gripping members of a forceps is provided with teeth 3, 4 which are arranged at the same angle on each side of the imaginary median line 2 of said instrument. The angle which the teeth make with said imaginary line may be of any degree, but an acute angle is preferable. The teeth 3 on one side of said line 2 are staggered with relation to the teeth 4 on the other side of said line 2 and start at exactly half-way between the teeth of the opposite side. The other gripping member of the forceps is provided with a gripping part 1 of an identical construction.

When the two identical parts of the forceps cast according to the invention from plastics or metal, or stamped from metal, are assembled, with one part facing the other, the teeth 4 of one part will matingly engage in the interstices or grooves 5 between the teeth 3 of the other part, thereby imparting a tight gripping action to the forceps.

-4-   **0176351**

CLAIMS:

1.   Gripper means on each pair of gripping parts (1) of a gripping tool, characterised in that the teeth (3) and the interstices (5) on one side of an imaginary longitudinal median line (2) form a surface relief pattern which is substantially duplicated by the teeth (4) and interstices (6) on the other side of the line (2), but in the reverse sense, so that imaginary folding of the whole surface along the median line (2) will result in a substantial mating fit between teeth (3, 4) on one side of the line (2) and interstices (5, 6) on the other side of the line (2).

2.   Gripper means as claimed in claim 1, characterised in that the teeth (3, 4) and the interstices (5, 6) cover substantially the whole of the inside surface of the gripping part (1).

3.   Gripper means as claimed in claim 1 or claim 2, characterised in that the teeth (3, 4) and the interstices (5, 6) on each side of the median line (2) are arranged in straight, parallel rows.

4.   Gripper means on the gripping parts of a gripping tool (1) comprising teeth (3, 4) which extend at the same angle on the two sides of an imaginary longitudinal median line (2) of each part, the teeth (3) on one side being staggered relative to those (4) on the other.

5.   Gripper means as claimed in claim 4, wherein the said angle is of any suitable degree.

6.   Gripper means as claimed in claim 6, wherein the said angle is acute.

7.   Gripper means as claimed in any one of the preceding claims, wherein the tool is of molded plastics or of stamped or molded metal.

8.   A gripping tool having gripping means as claimed in any one of the preceding claims.

9.   A gripping tool as claimed in claim 8 in the form of a forceps or like medical instrument.

10.   A gripping tool as claimed in claim 8 or claim 9, characterised in that its gripping parts (1) are of substantially identical construction.

0176351